(19) Europäisches Patentamt
European Patent Office
Office européen des brevets

(11) Publication number: **0 091 502**
**A1**

(12) # EUROPEAN PATENT APPLICATION

(21) Application number: 82103114.3

(22) Date of filing: 13.04.82

(51) Int. Cl.³: **A 61 K 31/545**
A 61 K 31/535, A 61 K 9/02

(43) Date of publication of application:
19.10.83 Bulletin 83/42

(84) Designated Contracting States:
AT BE CH DE FR GB IT LI LU NL SE

(71) Applicant: Asahi Kasei Kogyo Kabushiki Kaisha
2-6, Dojimahama 1-chome Kita-ku
Osaka-shi Osaka 530(JP)

(72) Inventor: Yata, Noboru
17-6-302, Yoshishimahigashi 2-chome
Naka-ku Hiroshima-shi Hiroshima-ken(JP)

(74) Representative: Boeters, Hans Dietrich, Dr. et al,
Boeters, Bauer & Partner Thomas-Wimmer-Ring 14
D-8000 München 22(DE)

(54) **A pharmaceutical preparation of cephalosporin for rectal administration.**

(57) A pharmaceutical preparation for rectal administration which comprises an oleaginous base and, incorporated therein, a third generation cephalosporin, an amino acid and an ether type nonionic surfactant has been found to be not only very excellent in absorbability of the cephalosporin into the body, but also extremely moderate in tissue-damaging action.

EP 0 091 502 A1

This invention relates to a pharmaceutical preparation of cephalosporin for rectal administration. More particularly, the present invention is concerned with a pharmaceutical preparation for rectal administration which comprises an oleaginous base and, incorporated therein, a third generation cephalosporin, an amino acid and an ether type nonionic surfactant.

When hardly absorbable water-soluble medicines such as insulin and cefazolin are used for the systemic therapy, the administration mode is limited to injection. Even in the case of compounds which can be absorbed by oral administration, such as cephalexin and ampicillin, administration thereof to patients suffering from dysphagia or infants and babies encounters difficulties, and, therefore, the absorbed amounts of the medicines are often insufficient.

As the new administration mode capable of overcoming these difficulties, there has been proposed and examined a rectal administration method. However, if a medicinal compound alone is administered, no sufficient absorption can be attained, and it has been reported that only when a medicinal compound is administered in combination with a surfactant, especially an ether type nonionic surfactant, the absorbed amount of the medicinal compound is increased (see, for example, Japanese Patent Application Laid-Open Specification No. 72316/1973).

This ether type nonionic surfactant, however, is defective in that it has a strong tissue-damaging action and it cannot be directly put into practical use.

I have made researches with a view to developing a practical pharmaceutical preparation for rectal administration while eliminating the above defects of an ether type nonionic surfactant. As a result, I have found that when an amino acid is added to a mixture comprising a water-soluble medicine, an ether type nonionic surfactant and an oleaginous base, the tissue-damaging action of the ether type nonionic surfactant can be drastically moderated and the absorbability of the medicine can be increased.

It has also been found that when a third generation cephalosporin which is excellent in antimicrobial activity is used as the water-soluble medicine in the above preparation, there are obtained good results in the tissue-damage test and the concentration-in-blood test. By the term "third generation cephalosporin" used herein is meant a cephalosporin having a resistance to a $\beta$-lactamase and an antimicrobial activity to not only gram-positive bacteria but also gram-negative bacteria. Accordingly, when the third generation cephalosporin is used in the above-mentioned pharmaceutical preparation and the resulting pharmaceutical preparation is applied to patients suffering from diseases caused by gram-negative bacteria or resistant bacteria, which diseases can hardly be cured by known water-soluble medicines, it is expected that good curative effects will be obtained while suppressing occurrence of side effects such as diarrhoea by the action of an amino acid incorporated in the pharmaceutical preparation. Moreover, it has been found that this pharmaceutical preparation shows satisfactory results in the

test for remedy of infectious diseases. The present invention has been made, based on such novel findings.

As the third generation cephalosporin (hereinafter often referred to only as "cephalosporin") to be used in the present invention, there may advantageously be employed those of the general formula (I) and water-soluble non-toxic salts thereof:

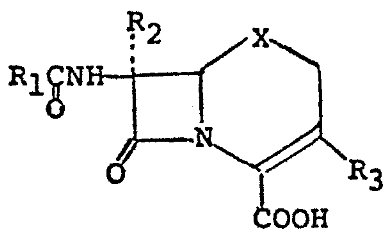

(I)

wherein $R_1$ stands for an aromatic ring-substituted methyl group, an α-substuted, aromatic ring-substituted methyl group, an α-substituted, aromatic heterocycle-substituted methyl group or a substituted or unsubstituted alkyl group, said aromatic ring and said aromatic heterocycle each being substituted or unsubstituted, $R_2$ stands for a hydrogen atom or a methoxy group, $R_3$ stands for a hydrogen atom, a substituted or unsubstituted heterocycle-substituted thiomethyl group, an acetoxymethyl group, an aminocarbonyloxymethyl group or a substituted or unsubstituted heterocycle-substituted methyl group, and X stands for S or O.

Specific examples of the third generation cephalosporin represented by the general formula (I) include

compounds of the following formulae (II) to (XIV):

Cefuroxime

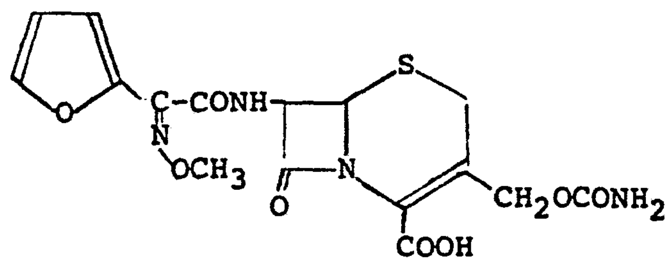

(II) ,

Cefoperazone

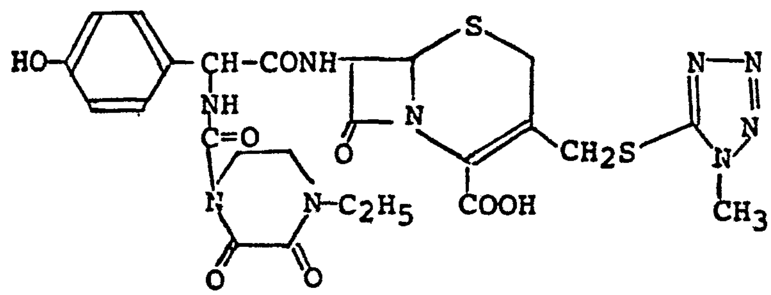

(III),

Cefotaxime

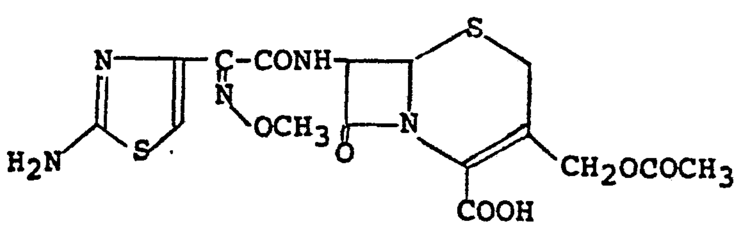

(IV) ,

Moxalactam

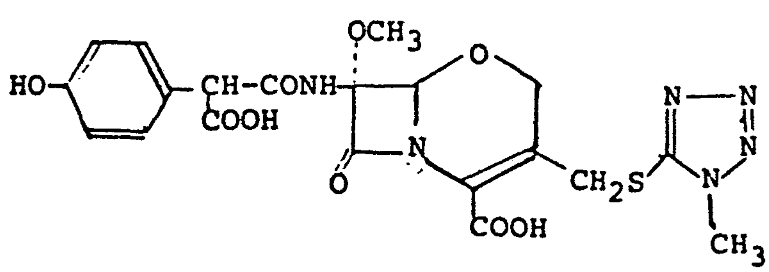

(V) ,

Ceftizoxime

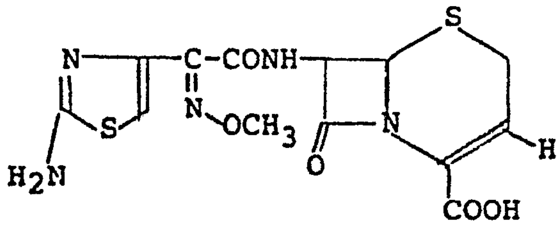

(VI) ,

Cefmenoxime

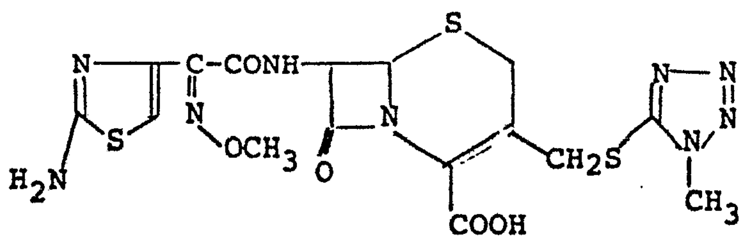

(VII),

T-1982

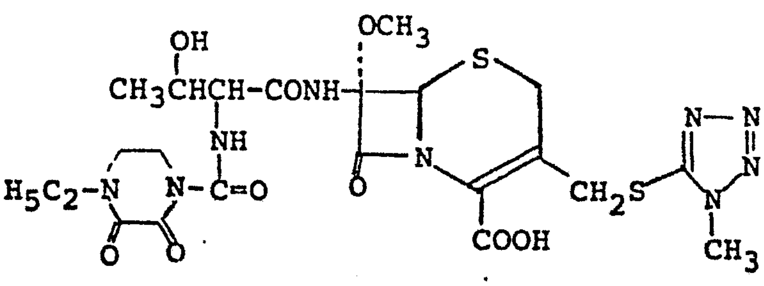

(VIII),

MT-141

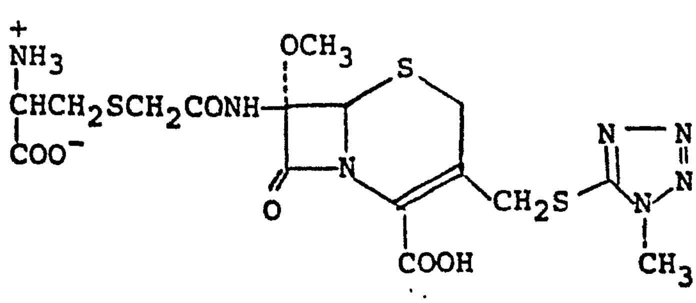

(IX) ,

Cefpiramide

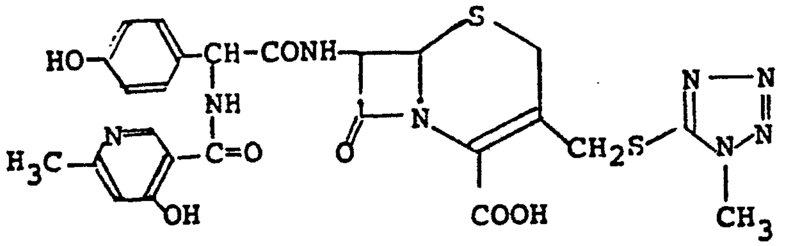

(X),

AC-1370

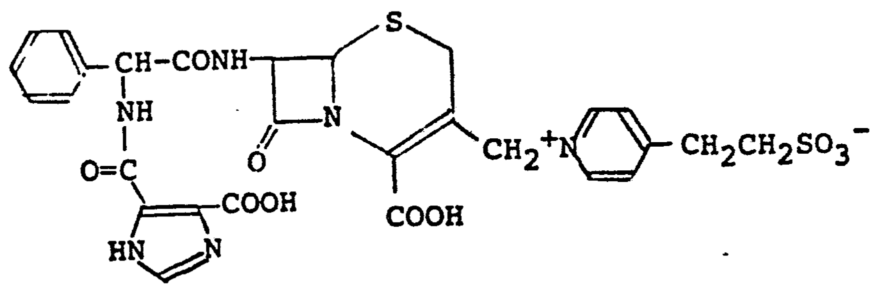

(XI),

Cefutazidime

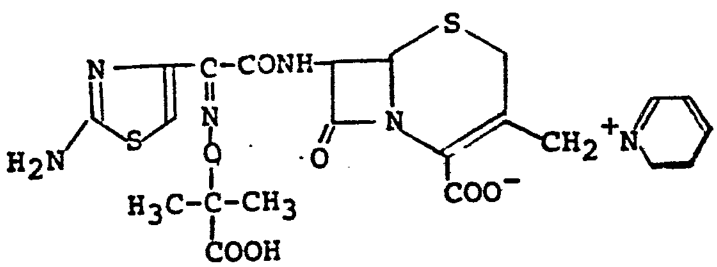

(XII),

Ceftriaxone

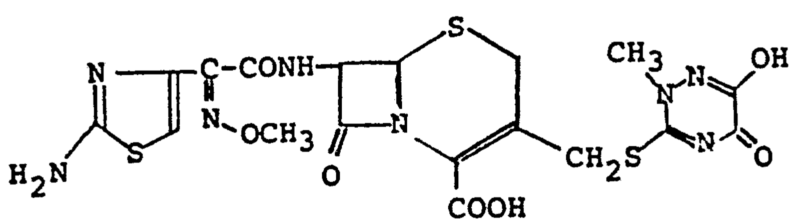

(XIII),

and

Cefolanide

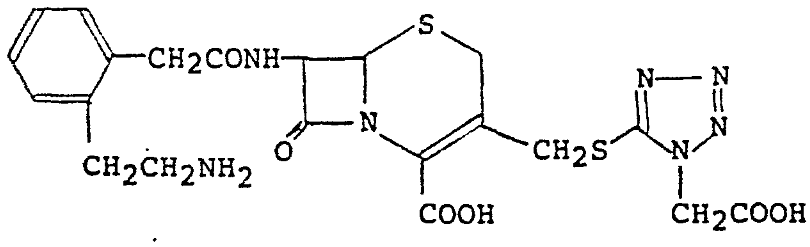

(XIV)

As examples of the above-mentioned water-soluble non-toxic salt, there can be mentioned sodium salts, potassium salts and hydrochlorides of the foregoing compounds.

The caphalosporin as the active ingredient may be incorporated in an amount of 2.5 to 50 % by weight, preferably 5 to 40 % by weight, based on the total weight of the pharmaceutical preparation.

In the present invention, as the ether type nonionic surfactant, there can be mentioned at least one member selected from polyoxyethylene alkyl ethers, polyoxyethylene alkylaryl ethers and polyoxyethylene-polyoxypropylene alkyl ethers. Other nonionic surfactants such as polyoxyethylene sorbitan fatty acid esters and polyoxyethylene hardened castor oils are inferior to the ether type nonionic surfactant in the effect of improving the absorbability of the medicine, though they are advantageous in that the tissue-damaging action is slightly weaker than that of the ether type nonionic surfactant. Accordingly, from the practical viewpoint, use of these other nonionic surfactants is not advantageous.

As the polyoxyethylene alkyl ethers, there can be

mentioned, for example, polyoxyethylene lauryl ether, polyoxyethylene oleyl ether, polyoxyethylene cetyl ether, polyoxyethylene stearyl ether and polyoxyethylene butyl ether. As the polyoxyethylene alkylaryl ethers, there can be mentioned, for example, polyoxyethylene nonylphenyl ether and polyoxyethylene octylphenyl ether. As the polyoxyethylene-polyoxypropylene alkyl ehters, there can be mentioned, for example, polyoxyethylene-polyoxypropylene cetyl ether and polyoxyethylene-polyoxypropylene behenyl ether. In these ethers, it is preferred that the degree of polymerization of the polyoxyethylene (or polyoxyethylene-polyoxypropylene) be 5 to 20 and the carbon number of the alcohol moiety be 2 to 22, and it is especially preferred that the degree of polymerization of the polyoxyethylene (or polyoxyethylene-polyoxypropylene) be 5 to 15, the carbon number of the chain portion be 7 to 18 and the HLB value be 7 to 15. The ether type nonionic surfactant may be incorporated in an amount of 0.05 to 10 % by weight, preferably 0.3 to 5 % by weight, based on the total weight of the pharmaceutical preparation.

As the amino acid, there can be mentioned neutral amino acids exclusive of glycine, basic amino acids and acidic amino acids. They may be used alone or in mixture. It has been found that glycine has no effect of weakening the tissue-damaging action of the ether type nonionic surfactant. A crude amino acid mixture obtained by the hydrolysis of a protein or peptide may also be used as the amino acid in the present invention. Specific examplese of the amino acid

include alanine, valine, leucine, isoleucine, serine, threonine, aspartic acid, asparagine, glutamic acid, glutamine, phenylalanine, tyrosine, tryptophane, proline, hydroxyproline, alginine, lysine, histidine, methionine, cystine and cysteine. They may be employed alone or in mixture. Among these amino acids, aspartic acid, glutamic acid, asparagine, glutamine, methionine, alginine, lysine and histidine are especially preferred. They may be employed in a free form. But, the basic amino acids may be used in the form of a hydrochloride or sulfate, and the acidic amino acids may be used in the form of a water-soluble non-toxic salt such as a sodium salt or potassium salt. The effect of weakening of tissue-damaging action of the ether type nonionic surfactant by the amino acid is not changed according to the kind of the amino acid, that is, the effect is not changed whether the amino acid used is a neutral amino acid, a basic amino or an acidic amino acid. However, the basic amino acid is excellent in the effect of improving the absorbability of the water-soluble medicine. The amino acid may be incorporated in an amount of 0.5 to 20 % by weight, preferably 1 to 10 % by weight, based on the total weight of the pharmaceutical preparation.

As the oleaginous base to be used in the present invention, there may be employed any of bases customarily used for the production of ointments and suppositories. For example, there can be mentioned oils and fats such as peanut oil, coconut oil, olive oil, soybean oil, linseed oil, cotton

seed oil, sesame oil, corn oil, rice bran oil, camellia oil, cacao butter, lard, wool fat and beef tallow; these oils and fats modified by hydrogenation, acetylation or fractional extraction; glycerol esters of fatty acids having 6 to 30 carbon atoms (such as Miglyol® and Witepsol® manufactured and sold by Dynamit Nobel, W. Germany, SB® manufactured and sold by Kanegafuchi Chemical Industry Co., Ltd., Japan and Panacete® manufactured and sold by Nippon Oil & Fats Co., Ltd., Japan; and esters of fatty acids having 6 to 30 carbon atoms with alcohols having 2 to 8 carbon atoms (such as isopropyl myristate). These oils and fats may be used alone or in mixture. In general, as the pharmaceutical preparation for rectal administration, those which are solid at room temperature and are adapted to be molten at the body temperature are conveniently used, and, therefore, a glycerol ester of a fatty acid having 10 to 30 carbon atoms, cacao butter or the like is especially preferred as the oleaginous base.

The pharmaceutical preparation for rectal administration according to the present invention can be produced by blending an oleaginous base and an ether type nonionic surfactant, followed by melting to obtain a melt; homogeneously dispersing an amino acid and a cephalosporin into the melt; and subjecting the resulting dispersion to shaping or preparation according to the known method adopted for the production of ointments or suppositories. If necessary, additives such as stabilizers and antiseptics may be added.

The pharmaceutical preparation for rectal administration

of the present invention may be not only in the form of an anal suppository which is solid at room temperature and is adapted to be molten at the body temperature, but also in such a form as is of an ointment or enema which has been prepared by dispersing the cephalosporin, ether type nonionic surfactant and amino acid in a liquid oil or fat and can be administered by means of a soft capsule for rectal administration or an injector for rectal administration.

The pharmaceutical preparation of the present invention may generally be administered in an amount of 500 mg to 10 g, preferably 1 to 5 g per day for adults in terms of amount of a cephalosporin as the active ingredient, although the dosage should be determined by skilled physicians taking into consideration the ages and weight of patients, kinds and severities of disorders and other factors.

The present invention will now be described with reference to Experiments illustrating the absorbability, tissue-damaging action and infeoted animal-curing activity of the pharmaceutical preparation for rectal administration according to the present invention and Examples illustrating the process for the production of the pharmaceutical preparation for rectal administration. However, it should be noted that the present invention is by no means limited by these Experiments and Examples.

Experiment 1

The following animal tests were conducted with Wister male rats (body weight: about 200 g) in order to investigate a tissue-damaging action caused by the administration of preparations of the present invention and comparative preparations.

A preparation as shown in Table 1 was administered to the rectum of the rat in an amount of 30 mg of cephalosporin per kilogram of rat which rat had been subjected to fast for 24 hours. When the preparation was solid at room temperature, it was administered in the form of an anal suppository. On the other hand, when the preparation was either an ointment or liquid, it was administered by means of a small syringe. After administration of the preparation, the anus of the rat was sealed by an instant adhesive, Aron Alpha (manufactured and sold by Toa Gosei Chemical Industry Company, Ltd., Japan) so as to prevent the preparation from leaking. The rat was killed by venesection four hours after the administration. Immediately after killing the rat, the rectum of the rat was cut off and examined with the naked eye. The number of rats in which congestion and festering were observed in the rectum was counted and the incidence was calculated. The results obtained are shown in Table 1.

Table 1

Results of tissue damage test (dose: 30 mg as cephalosporin per kilogram of rat)

| | Run No. | Preparation No. | Ingredients: Cephalosporin, wt% | Ingredients: Amino acid, wt% | Ingredients: Nonionic surfactant, wt% | Ingredients: Oleaginous base, wt% | Number of rats tested | Rectum damage: Festering: Number of rats | Festering: Incidence (%) | Congestion: Number of rats | Congestion: Incidence (%) | Total: Number of rats damaged | Total: Incidence (%) |
|---|---|---|---|---|---|---|---|---|---|---|---|---|---|
| Comparative preparations | 1 | 1 | Cefuroxime sodium, 7.5 | None | Polyoxyethylene lauryl ether, 1.0 | SB-AM, 91.5 | 25 | 15 | 60.0 | 17 | 68.0 | 20 | 80.0 |
| | 2 | 2 | Cefuroxime sodium, 7.0 | None | Polyoxyethylene lauryl ether, 0.5 | SB-AM, 92.5 | 6 | 4 | 66.7 | 4 | 66.7 | 4 | 66.7 |
| | 3 | 3 | Cefuroxime sodium, 7.0 | None | Polyoxyethylene cetyl ether, 1.0 | SB-AM, 92.0 | 6 | 3 | 50.0 | 4 | 66.7 | 5 | 83.3 |
| | 4 | 4 | Cefotaxime sodium, 6.0 | None | Polyoxyethylene lauryl ether, 1.0 | SB-AM, 93.0 | 6 | 3 | 50.0 | 5 | 83.3 | 5 | 83.3 |
| Preparations of the present invention | 5 | 5 | Cefuroxime sodium, 7.5 | Arginine hydrochloride, 3.5 | Polyoxyethylene lauryl ether, 1.0 | SB-AM, 88.0 | 30 | 3 | 10.0 | 4 | 13.3 | 4 | 13.3 |
| | 6 | 6 | Cefuroxime sodium, 6.0 | ysine hydrochloride, 4.0 | Polyoxyethylene lauryl ether, 1.0 | SB-AM, 89.0 | 6 | 1 | 16.7 | 1 | 16.7 | 1 | 16.7 |
| | 7 | 7 | Cefuroxime sodium, 7.0 | Methionine, 7.0 | Polyoxyethylene lauryl ether, 0.5 | SB-AM, 85.5 | 6 | 1 | 16.7 | 1 | 16.7 | 1 | 16.7 |
| | 8 | 8 | Cefuroxime sodium, 7.0 | Valine, 10.0 | Polyoxyethylene cetyl ether, 1.0 | SB-AM, 82.0 | 6 | 1 | 16.7 | 1 | 16.7 | 1 | 16.7 |

(to be continued)

( continued)

| | | | | | | | | | | | | | |
|---|---|---|---|---|---|---|---|---|---|---|---|---|---|
| Preparations of the present invention | 9 | 9 | Cefotaxime sodium, 6.0 | Arginine hydrochloride, 3.5 | Polyoxyethylene lauryl ether, 0.5 | SB-AM, 90.0 | 6 | 0 | 0 | 1 | 16.7 | 1 | 16.7 |
| | 10 | 10 | Cefotaxime sodium, 5.0 | Hydroxylysine hydrochloride, 5.0 | Polyoxyethylene lauryl ether, 1.0 | SB-AM, 89.0 | 6 | 0 | 0 | 0 | 0 | 0 | 0 |
| | 11 | 11 | Cefoperazone sodium, 10.0 | Hydroxyproline, 7.0 | Polyoxyethylene lauryl ether, 2.0 | SB-AM, 81.0 | 6 | 0 | 0 | 0 | 0 | 0 | 0 |
| | 12 | 12 | Moxalactam sodium, 6.0 | Lysine hydrochloride, 6.0 | Polyoxyethylene lauryl ether, 1.0 | Witepsol H-15, 87.0 | 6 | 0 | 0 | 1 | 16.7 | 1 | 16.7 |
| | 13 | 13 | Ceftizoxime sodium, 10.0 | Asparagine, 8.0 | Polyoxyethylene cetyl ether, 2.0 | Witepsol H-15, 80.0 | 6 | 1 | 16.7 | 1 | 16.7 | 1 | 16.7 |
| | 14 | 14 | Cefmenoxime hydrochloride, 10.0 | Lysine hydrochloride, 10.0 | Polyoxyethylene cetyl ether, 3.0 | Witepsol H-15, 77.0 | 6 | 0 | 0 | 0 | 0 | 1 | 16.7 |
| | 15 | 15 | T-1982, 7.0 | Lysine hydrochloride 7.0 | Polyoxyethylene cetyl ether, 2.0 | Witepsol H-15, 84,0 | 6 | 0 | 0 | 0 | 0 | 0 | 0 |
| Comparative preparation | 16 | 16 | MT-141, 10.0 | None | Polyoxyethylene-polyoxypropylene cetyl ether, 5.0 | Isocacao butter, 85.0 | 12 | 8 | 66.7 | 10 | 83.3 | 10 | 83.3 |
| ...ra... of the present invention | 17 | 17 | MT-141, 10.0 | Proline, 10.0 | Polyoxyethylene-polyoxypropylene cetyl ether, 5.0 | Isocacao butter, 75.0 | 6 | 0 | 0 | 1 | 16.7 | 1 | 16.7 |

( continued )

| | | | | | | | | | | | | | |
|---|---|---|---|---|---|---|---|---|---|---|---|---|---|
| Comparative preparations | 18 | 18 | Cefpiramide sodium, 10.0 | None | Polyoxyethylene stearyl ether, 2.0 | Miglyol 812, 88.0 | 8 | 3 | 37.5 | 5 | 62.5 | 7 | 87.5 |
| | 19 | 19 | AC-1370, 5.0 | None | Polyoxyethylene nonylphenyl ether, 2.0 | Peanut oil, 93.0 | 8 | 5 | 62.5 | 8 | 100 | 8 | 100 |
| | 20 | 20 | Cefutazidime, 15.0 | None | Polyoxyethylene octylphenyl ether, 5.0 | Panacete 810, 80.0 | 10 | 5 | 50 | 7 | 70 | 8 | 80 |
| Preparations of the present invention | 21 | 21 | Cefpiramide sodium, 10.0 | Isolysine, 10.0 | Polyoxyethylene stearyl ether, 2.0 | Miglyol 812, 78.0 | 6 | 1 | 16.7 | 1 | 16.7 | 1 | 16.7 |
| | 22 | 22 | Ceftriaxone sodium, 8.0 | Sodium glutamate, 7.0 | Polyoxyethylene lauryl ether, 1.0 | Corn oil, 84.0 | 6 | 0 | 0 | 0 | 0 | 0 | 0 |
| | 23 | 23 | AC-1370, 5.0 | Glutamine, 5.0 | Polyoxyethylene oleyl ether, 1.0 | Soybean oil, 89.0 | 6 | 0 | 0 | 0 | 0 | 0 | 0 |
| | 24 | 24 | AC-1370, 5.0 | Histidine, 5.0 | Polyoxyethylene nonylphenyl ether, 2.0 | Peanut oil, 88.0 | 6 | 1 | 16.7 | 0 | 0 | 1 | 16.7 |
| | 25 | 25 | Moxalactam sodium, 15.0 | Sodium as-paraginate, 15.0 | Polyoxyethylene nonylphenyl ether, 5.0 | Panacete 810, 65.0 | 6 | 0 | 0 | 1 | 16.7 | 1 | 16.7 |
| | 26 | 26 | MT-141, 8.0 | Threonine, 7.0 | Polyoxyethylene cetyl ether, 2.0 | Olive oil, 83.0 | 6 | 1 | 16.7 | 0 | 0 | 1 | 16.7 |

(to be continued)

( continued )

| | | | | | | | | | | | | |
|---|---|---|---|---|---|---|---|---|---|---|---|---|
| 27 | 27 | Cefoperazone sodium, 15.0 | Arginine hydrochloride, 10.0 | Polyoxyethylene oleyl ether, 3.0 | Miglyol 812, 72.0 | 6 | 0 | 0 | 0 | 0 | 0 | 0 |
| 28 | 28 | Cefolanide sodium, 7.0 | Tyrosine, 7.0 | Polyoxyethylene octylphenyl ether, 2.0 | Miglyol 812, 84.0 | 6 | 1 | 16.7 | 0 | 0 | 1 | 16.7 |

Experiment 2

The following animal tests were conducted with Wister male rats (body weight: about 200 g) in order to evaluate the absorbability of the preparations of the present invention and comparative preparations as shown in Table 2.

A preparation as shown in Table 2 was administered to the rectum of the rat in an amount of 30 mg of cephalosporin per kilogram of rat which rat had been subjected to fast for 24 hours. When the preparation was solid at room temperature, it was administered in the form of an anal suppository. On the other hand, when the preparation was either an ointment or liquid, it was administered by means of a small syringe. After administration of the preparation, the anus of the rat was sealed by an instant adhesive, Aron Alpha (manufactured and sold by Toa Gosei Chemical Industry Company, Ltd.) so as to present the preparation from leaking.

Blood was collected at predetermined intervals from the jugular vein of the rat. The collected blood was subjected to centrifugation at 3,000 rpm for 10 minutes to obtain plasma as a supernatant fluid. The concentration of cephalosporin in blood was determined according to the biological assay. That is, *Bacillus subtilis* was used as the bacillus and was cultured at 37°C for 16 to 20 hours according to the paper disc method and the biological assay was then carried out. With respect to the paper disc method, reference may be made to "Saikingaku Jisshu Teiyo (Outline of Bacteriological Experiments)" pages 376 to 377, published by Maruzen Co., Ltd., Japan. The test results obtained are shown in Table 2.

**Table 2**

**Results of test on concentration in blood (dose: 30 mg as cephalosporin per kilogram of rat)**

| | Run No. | Preparation No. | Ingredients | | | | Concentration in blood (μg/ml) | | | | | | |
|---|---|---|---|---|---|---|---|---|---|---|---|---|---|
| | | | Cephalosporin, wt% | Amino acid, wt% | Nonionic surfactant, wt% | Oleaginous base, wt% | 10 min | 20 min | 40 min | 60 min | 80 min | 120 min | 180 min |
| Comparative preparations | 29 | 29 | Cefuroxime sodium, 7.5 | None | None | SB-AM, 92.5 | 0.8 | 0.6 | 0.6 | 0.4 | – | – | – |
| | 30 | 1 | Cefuroxime sodium, 7.5 | None | Polyoxyethylene lauryl ether, 1.0 | SB-AM, 91.5 | 12.0 | 9.2 | 5.2 | 2.3 | 1.2 | 1.0 | – |
| | 31 | 4 | Cefotaxime sodium, 6.0 | None | Polyoxyethylene lauryl ether, 1.0 | SB-AM, 93.0 | 14.1 | 13.8 | 8.1 | 2.5 | 1.0 | – | – |
| Preparations of the present invention | 32 | 5 | Cefuroxime sodium, 7.5 | Arginine hydrochloride, 3.5 | Polyoxyethylene lauryl ether, 1.0 | SB-AM, 88.0 | 13.2 | 17.3 | 14.1 | 7.0 | 4.2 | – | – |
| | 33 | 6 | Cefuroxime sodium, 6.0 | Lysine hydrochloride, 4.0 | Polyoxyethylene lauryl ether, 1.0 | SB-AM, 89.0 | 17.2 | 22.1 | 12.1 | 7.2 | 3.8 | – | – |
| | 34 | 8 | Cefuroxime sodium, 7.0 | Valine, 10.0 | Polyoxyethylene cetyl ether, 1.0 | SB-AM, 82.0 | 14.8 | 12.7 | 8.0 | 5.1 | 2.8 | – | – |
| | 35 | 10 | Cefotaxime sodium, 5.0 | Hydroxylysine hydrochloride, 5.0 | Polyoxyethylene lauryl ether, 1.0 | SB-AM, 89.0 | 15.1 | 25.0 | 15.0 | 6.8 | 2.5 | 2.5 | 2.0 |
| | 36 | 12 | Moxalactam sodium, 6.0 | Lysine hydrochloride, 6.0 | Polyoxyethylene lauryl ether, 1.0 | Witepsol H-15, 87.0 | 22.0 | 31.0 | 22.2 | 15.4 | 9.2 | 3.8 | 3.0 |

(to be continued)

( continued )

| | | | | | | | | | | | | | |
|---|---|---|---|---|---|---|---|---|---|---|---|---|---|
| | 37 | 14 | Cefmenoxime hydrochloride, 10,0 | Lysine hydrochloride 10.0 | Polyoxyethylene cetyl ether, 3.0 | Witepsol H-15, 77.0 | 15.2 | 18.4 | 10.0 | 4.2 | 2.1 | 2.0 | 1.8 |
| Comparative preparation | 38 | 16 | MT-141, 10.0 | None | Polyoxyethylene-polyoxypropylene cetyl ether, 5.0 | Isocacao butter, 85.0 | 10.2 | 12.4 | 5.2 | 1.5 | 1.0 | — | — |
| Preparation of the present invention | 39 | 17 | MT-141, 10.0 | Proline, 10.0 | Polyoxyethylene-polyoxypropylene cetyl ether, 5.0 | Isocacao butter 75.0 | 12.3 | 14.5 | 9.2 | 3.4 | 1.2 | 0.5 | — |
| Comparative composition | 40 | 18 | Cefpiramide sodium, 10.0 | None | Polyoxyethylene stearyl ether, 2.0 | Miglyol 812, 88.0 | 10.2 | 9.2 | 4.8 | 3.2 | 1.2 | — | — |
| Preparation of the present invention | 41 | 21 | Cefpiramide sodium, 10.0 | Isoleucine 10.0 | Polyoxyethylene stearyl ether, 2.0 | Miglyol 812, 78.0 | 12.4 | 14.5 | 9.1 | 6.8 | 2.1 | 1.0 | 0.5 |
| Comparative preparations | 42 | 19 | AC-1370, 5.0 | None | Polyoxyethylene nonylphenyl ether, 2.0 | Peanut oil, 93.0 | 12.3 | 13.5 | 10.2 | 6.9 | 2.0 | 1.0 | — |
| | 43 | 20 | Moxalactam, 15.0 | None | Polyoxyethylene octylphenyl ether, 5.0 | Panacete 810, 80.0 | 13.4 | 20.0 | 12.8 | 4.8 | 2.0 | 1.5 | — |

(to be continued)

( continued )

| | | | | | | | | | | | | | | |
|---|---|---|---|---|---|---|---|---|---|---|---|---|---|---|
| Preparations of the present invention | 44 | 24 | AC-1370, 5.0 | Histidine, 5.0 | Polyoxyethylene nonylphenyl ether, 2.0 | Peanut oil, 88.0 | 20.2 | 25.4 | 11.2 | 5.7 | 3.8 | 2.1 | 1.0 |
| | 45 | 25 | Moxalactam sodium, 15.0 | Sodium asparaginate, 15.0 | Polyoxyethylene octylphenyl ether, 5.0 | Panacete 810, 65.0 | 21.2 | 32.3 | 14.8 | 10.0 | 4.2 | 2.0 | 1.6 |

Experiment 3

The animal tests with domestic male rabbits (body weight: about 3 kg) were carried out in the same manner as in Experiment 2, except that the anus of the rabbit was sealed by a clip after the preparation as shown in Table 3 was administered to the rectum of the rabbit and that blood was collected from the aural vein of the rabbit. The results obtained are shown in Table 3.

**Table 3**

Results of test on concentration in blood (dose: 15 mg as cephalosporin per kilogram of rabbit)

| | Run No. | Preparation No. | Ingredients: Cephalosporin, wt% | Ingredients: Amino acid, wt% | Ingredients: Nonionic surfactant, wt% | Ingredients: Oleaginous base, wt% | Concentration in blood (μg/ml): 10 min | 20 min | 40 min | 60 min | 80 min | 120 min | 180 min |
|---|---|---|---|---|---|---|---|---|---|---|---|---|---|
| Comparative preparation | 46 | 2 | Cefuroxime sodium, 7.0 | None | Polyoxyethylene lauryl ether, 0.5 | SB-AM, 92.5 | 8.2 | 9.0 | 4.8 | 2.3 | 1.0 | 0.5 | — |
| | 47 | 30 | Cefotaxime sodium, 6.0 | None | Polyoxyethylene lauryl ether, 0.5 | SB-AM, 93.5 | 8.1 | 12.3 | 16.0 | 10.1 | 7.8 | 5.2 | 2.7 |
| | 48 | 31 | Ceftizoxime sodium, 10.0 | None | Polyoxyethylene cetyl ether, 0.5 | SB-AM, 89.5 | 6.4 | 8.0 | 6.4 | 4.2 | 4.0 | 1.3 | — |
| Preparations of the present invention | 49 | 7 | Cefuroxime sodium, 7.0 | Methionine, 7.0 | Polyoxyethylene lauryl ether, 0.5 | SB-AM, 85.5 | 16.0 | 19.1 | 7.8 | 4.2 | 2.6 | 1.0 | 1.0 |
| | 50 | 9 | Cefotaxime sodium, 6.0 | Arginine hydrochloride, 3.5 | Polyoxyethylene lauryl ether, 0.5 | SB-AM, 90.0 | 17.2 | 18.5 | 8.0 | 4.0 | 2.3 | 1.0 | — |
| | 51 | 11 | Cefoperazone sodium, 10.0 | Hydroxyproline, 7.0 | Polyoxyethylene lauryl ether, 2.0 | SB-AM, 81.0 | 16.4 | 21.0 | 9.2 | 4.3 | 2.3 | 1.0 | 0.5 |
| | 52 | 13 | Ceftizoxime sodium, 10.0 | Asparagine, 8.0 | Polyoxyethylene cetyl ether, 2.0 | Witepsol H-15, 80.0 | 16.0 | 20.1 | 7.5 | 3.8 | 2.0 | 1.2 | 0.8 |
| | 53 | 15 | T-1982, 7.0 | Lysine hydrochloride, 7.0 | Polyoxyethylene cetyl ether, 2.0 | Witepsol H-15, 84.0 | 20.4 | 23.1 | 16.4 | 12.4 | 6.2 | 5.8 | 4.0 |

(to be continued)

( continued )

| | | | | | | | | | | | | | |
|---|---|---|---|---|---|---|---|---|---|---|---|---|---|
| Comparative preparations | 54 | 32 | Ceftriaxone sodium, 8.0 | None | Polyoxyethylene lauryl ether, 1.0 | Corn oil, 91.0 | 12.4 | 18.0 | 7.9 | 4.5 | 2.3 | 1.2 | 1.0 |
| | 55 | 33 | AC-1370, 5.0 | None | Polyoxyethylene oleyl ether, 1.0 | Soybean oil, 94.0 | 14.3 | 20.2 | 11.3 | 4.6 | 2.3 | 2.0 | 1.6 |
| Preparations of the present invention | 56 | 22 | Ceftriaxone sodium, 8.0 | Sodium glutamate, 7.0 | Polyoxyethylene lauryl ether, 1.0 | Corn oil, 84.0 | 15.3 | 20.0 | 10.5 | 4.0 | 3.0 | 2.2 | 2.0 |
| | 57 | 23 | AC-1370, 5.0 | Glutamine, 5.0 | Polyoxyethylene oleyl ether, 1.0 | Soybean oil, 89.0 | 20.0 | 25.4 | 17.2 | 11.0 | 3.2 | 2.5 | 1.7 |
| Comparative preparations | 58 | 34 | Cefuroxime sodium, 8.0 | None | Polyoxyethylene cetyl ether, 2.0 | Olive oil, 90.0 | 13.5 | 18.8 | 7.9 | 3.0 | 1.5 | 0.6 | — |
| | 59 | 35 | Cefoperazone sodium, 15.0 | None | Polyoxyethylene oleyl ether, 3.0 | Miglyol 812, 82.0 | 12.0 | 20.5 | 10.6 | 6.4 | 3.8 | 2.0 | 1.5 |
| Preparations of the present invention | 60 | 26 | MT-141, 8.0 | Threonine, 7.0 | Polyoxyethylene cetyl ether, 2.0 | Olive oil, 83.0 | 23.2 | 26.0 | 11.3 | 3.2 | 2.0 | 1.1 | — |
| | 61 | 27 | Cefoperazone sodium, 15.0 | Arginine hydrochloride, 10.0 | Polyoxyethylene oleyl ether, 3.0 | Miglyol 812, 72.0 | 17.9 | 25.6 | 12.7 | 8.0 | 5.4 | 4.2 | 2.0 |

Experiment 4

The following animal tests were conducted with ddY male mice (body weight: about 20 g) in order to evaluate the efficacy of the present preparations and comparative preparations.

A suspension of Serratia marcescens (T-55) containing 3% by weight of Mucin (manufactured and sold by Nakarai Chemical Ltd., Japan) was administered to a mouse by means of intra-abdominal ingection. After one hour of the injection, a preparation as shown in Table 4 was administered to the rectum of the mouse. When the preparation was solid at room temperature, it was administered in the form of an anal suppository. On the other hand, the preparation was either an ointment or liquid at room temperature, it was administered a depth of about 3 cm by means of a small syringe. MIC (minimum inhibitory concentration) and $ED_{50}$ (minimum effective dose) were determined to evaluate the efficacy of the present preparations and comparative preparations. The results obtained are shown in Table 4.

**Table 4**

Results of treatment test of animal infected with bacillus
(dose: 30 mg as cephalosporin per kilogram of mouse)

| | Run No. | Preparation No. | Ingredients | | | | MIC (minimum inhibitory concentration) [μg/ml] | | Quantity of infected cells [cells/mouse] | $ED_{50}$ (minimum effective dose) [mg/mouse] |
|---|---|---|---|---|---|---|---|---|---|---|
| | | | Cephalosporin, wt% | Amino acid, wt% | Nonionic surfactant, wt% | Oleaginous base, wt% | $10^8$ | $10^6$ | | |
| Comparative preparations | 62 | 36 | Cephazolin sodium, 6.0 | Arginine hydrochloride, 3.5 | Polyoxyethylene lauryl ether, 0.5 | SB-AM, 90.0 | >100 | >100 | $3.0 \times 10^6$ | >32 |
| | 63 | 37 | Cephalexin sodium, 6.0 | Lysine hydrochloride, 6.0 | Polyoxyethylene lauryl ether, 1.0 | Witepsol H-15, 87.0 | >100 | >100 | $3.0 \times 10^6$ | >20 |
| Preparations of the present invention | 64 | 9 | Cefotaxime sodium, 6.0 | Arginine hydrochloride, 3.5 | Polyoxyethylene lauryl ether, 0.5 | SB-AM, 90.0 | 0.19 | 0.10 | $3.0 \times 10^6$ | 0.71 |
| | 65 | 12 | Moxalactam sodium, 6.0 | Lysine hydrochloride, 6.0 | Polyoxyethylene lauryl ether, 1.0 | Witepsol H-15, 87.0 | 0.39 | 0.20 | $3.0 \times 10^6$ | 0.022 |
| | 66 | 13 | Ceftizoxime sodium, 10.0 | Asparagine, 8.0 | Polyoxyethylene cetyl ether, 2.0 | Witepsol H-15, 80.0 | 0.05 | 0.39 | $3.0 \times 10^6$ | 0.045 |
| | 67 | 14 | Cefmenoxime hydrochloride, 7.0 | Lysine hydrochloride, 7.0 | Polyoxyethylene cetyl ether, 2.0 | Witepsol H-15, 84.0 | 0.39 | 0.09 | $3.0 \times 10^6$ | 0.001 |

Example 1

176 g of SB-AM (a higher fatty acid triglyceride manufactured and sold by Kanegafuchi Chemical Industry Co., Ltd., Japan) was molten at 40°C. The molten SB-AM was mixed with 2 g of polyoxyethylene lauryl ether as the nonionic surfactant. Then, 1.5 g of finely divided Cefuroxime sodium and 7 g of finely divided arginine hydrochloride were added to the mixture and dispersed therein with stirring. The resulting dispersion was filled in a plastic suppository container having a capacity of 2 g, taking care so that precipitation was not caused. contents of the container were solidified by air-cooling to obtain a suppository.

Examples 2 to 7

Substantially the same procedures as in Example 1 were repeated to prepare suppositories containing ingredients as shown in Table 5.

Table 5

| | Ingredients | | | |
|---|---|---|---|---|
| | Cephalosporin, wt% | Amino acid, wt% | Nonionic surfactant, wt% | Oleaginous base, wt% |
| Example 2 | Cefuroxime sodium, 6.0 | Lysine hydrochloride, 4.0 | Polyoxyethylene lauryl ether, 1.0 | SB-AM, 89.0 |
| Example 3 | Cefuroxime sodium, 7.0 | Methionine, 7.0 | Polyoxyethylene lauryl ether, 0.5 | SB-AM, 85.5 |
| Example 4 | Cefuroxime sodium, 7.0 | Valine, 10.0 | Polyoxyethylene cetyl ether, 1.0 | SB-AM, 82.0 |
| Example 5 | Cefotaxime sodium, 6.0 | Arginine hydrochloride, 3.5 | Polyoxyethylene lauryl ether, 0.5 | SB-AM, 90.0 |
| Example 6 | Cefotaxime sodium, 5.0 | Hydroxylysine hydrochloride, 5.0 | Polyoxyethylene lauryl ether, 1.0 | SB-AM, 89.0 |
| Example 7 | Cefoperazone sodium, 10.0 | Hydroxyproline, 7.0 | Polyoxyethylene lauryl ether, 2.0 | SB-AM, 81.0 |

Example 8

174 g of Witepsol H-15 (a higher fatty acid triglyceride manufactured and sold by Dynamit Nobel, West Germany) was molten at 45°C. The molten Witepsol H-15 was mixed with 2 g of polyoxyethylene lauryl ether as the nonionic surfactant. Then, 1.2 g of finely divided Moxalactam sodium and 1.2 g of lysine hydrochloride were added to the mixture and dispersed therein with stirring. The resulting dispersion was filled in a plastic suppository container having a capacity of 2 g, taking care so that precipitation was not caused. The contents of the container were solidified

by air-cooling to obtain a suppository.

Examples 9 to 11

Substantially the same procedures as in Example 8 were repeated to prepare suppositories containing ingredients as shown in Table 6.

Table 6

| | Ingredients | | | |
|---|---|---|---|---|
| | Cephalosporin, wt% | Amino acid, wt% | Nonionic surfactant, wt% | Oleaginous base, wt% |
| Example 9 | Ceftizoxime sodium, 10.0 | Asparagine, 8.0 | Polyoxyethylene cetyl ether, 2.0 | Witepsol H-15, 80.0 |
| Example 10 | Cefmenoxime hydrochloride, 10.0 | Leucine, 10.0 | Polyoxyethylene oleyl ether, 3.0 | Witepsol H-15, 77.0 |
| Example 11 | T-1982, 7.0 | Lysine hydrochloride, 7.0 | Polyoxyethylene cetyl ether, 2.0 | Witepsol H-15, 84.0 |

Example 12

150 g of isocacao butter was molten at 45°C. The molten isocacao butter was mixed with 10 g of polyoxyethylene-polyoxypropylene cetyl ether. Then, 20 g of finely divided Cefuroxime sodium and 20 g of finely divided proline were added to the mixture and dispersed therein with stirring. The resulting dispersion was filled in a plastic suppository container having a capacity of 2 g, taking care so that precipitation was not caused. The contents of the container were solidified by rapid cooling in ice water to obtain a suppository.

Example 13

156 g of Miglyol 812 (manufactured and sold by Dynamit Nobel, West Germany) was mixed with 4 g of polyoxyethylene stearyl ether. Then, 20 g of finely divided Cefpiramide sodium and 20 g of finely divided isoleucine were added to the mixture and dispersed therein with stirring. The resulting dispersion was filled in polyethylene tubes (capacity : 2 g) for rectal administration, taking care so that precipitation was not caused.

Examples 14 to 21

In the same manner as in Example 13, polyethylene tubes for rectal administration were filled with ingredients as shown in Table 7.

Table 7

| | Ingredient | | | |
|---|---|---|---|---|
| | Cephalosporin, wt% | Amino acid, wt% | Nonionic surfactant, wt% | Oleaginous base, wt% |
| Example 14 | Ceftriaxone sodium, 8.0 | Sodium glutamate, 7.0 | Polyoxyethylene lauryl ether, 1.0 | Corn oil, 84.0 |
| Example 15 | AC-1370, 5.0 | Glutamine, 5.0 | Polyoxyethylene oleyl ether, 1.0 | Soybean oil, 89.0 |
| Example 16 | AC-1370, 5.0 | Histidine, 5.0 | Polyoxyethylene nonylphenyl ether, 2.0 | Peanut oil, 88.0 |
| Example 17 | Moxalactam sodium, 15.0 | Sodium asparaginate, 15.0 | Polyoxyethylene octylphenyl ether, 5.0 | Panacete 810, 65.0 |
| Example 18 | MT-141, 8.0 | Threonine, 7.0 | Polyoxyethylene cetyl ether, 2.0 | Olive oil, 83.0 |

( to be continued)

(continued)

| | | | | |
|---|---|---|---|---|
| Example 19 | Cefoperazone sodium, 15.0 | Arginine hydrochloride, 10.0 | Polyoxyethylene oleyl ether, 3.0 | Miglyol 812, 72.0 |
| Example 20 | Cefolanide sodium, 7.0 | Tyrosine, 7.0 | Polyoxyethylene octylphenyl ether, 2.0 | Miglyol 812, 84.0 |
| Example 21 | Ceftriaxone sodium, 6.0 | Arginine hydrochloride, 3.5 | Polyoxyethylene lauryl ether, 0.5 | SB-AM, 90.0 |

Note: "Panacete 810" is a product manufactured and sold by Nippon Oil & Fats Co., Ltd., Japan

What is claimed is:

1. A pharmaceutical preparation for rectal administration which comprises an oleaginous base and, incorporated therein, a third generation cephalosporin, an amino acid and an ether type nonionic surfactant.

2. A pharmaceutical preparation according to claim 1, wherein said third generation cephalosporin is at least one member selected from compounds by the general formula (I) and water-soluble non-toxic salts thereof:

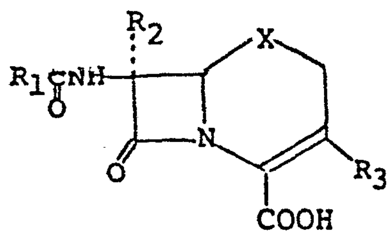

(I)

wherein $R_1$ stands for an aromatic ring-substituted methyl group, an α-substuted, aromatic ring-substituted methyl group, an α-substituted, aromatic heterocycle-substituted methyl group or a substituted or unsubstituted alkyl group, said aromatic ring and said aromatic heterocycle each being substituted or unsubstituted, $R_2$ stands for a hydrogen atom or a methoxy group, $R_3$ stands for a hydrogen atom, a substituted or unsubstituted heterocycle-substituted thiomethyl group, an acetoxymethyl group, an aminocarbonyloxymethyl group or a substituted or unsubstituted heterocycle-substituted methyl group, and X stands for S or O.

3. A pharmaceutical preparation according to claim 1, wherein said amino acid is at least one member selected from the group consisting of neutral amino acids exclusive of glycine, basic amino acids and water-soluble non-toxic salts thereof, and acidic amino acids and water-soluble non-toxic salts thereof.

4. A pharmaceutical preparation according to claim 1, said amino acid is incorporated in an amount of 0.5 to 20 % by weight based on the total weight of the pharmaceutical preparation.

5. A pharmaceutical preparation according to claim 1, wherein said ether type nonionic surfactant is at least one member selected from polyoxyethylene alkyl ethers, polyoxyethylene alkylaryl ethers and polyoxyethylene-polyoxypropylene alkyl ethers.

6. A pharmaceutical preparation according to claim 1, wherein said ether type nonionic surfactant is incorporated in an amount of 0.05 to 10% by weight based on the total weight of the pharmaceutical composition.

7. A pharmaceutical preparation according to claim 2, wherein said compounds include those of the formulae (II) to (XIV):

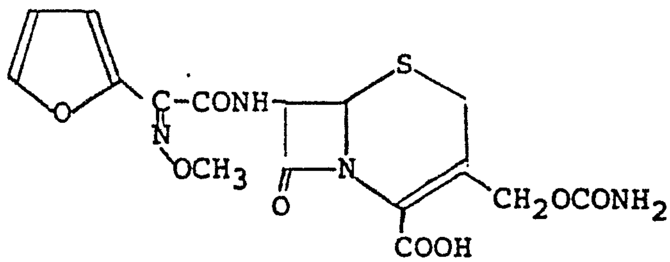

(II) ,

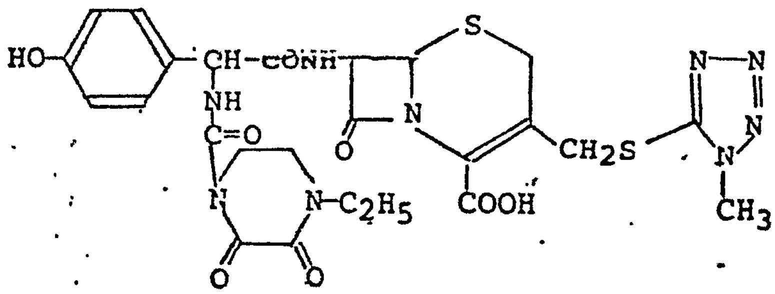

(III),

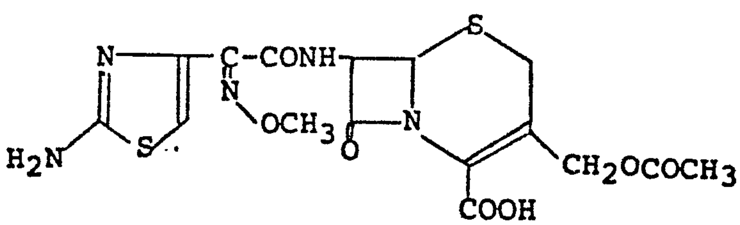

(IV) ,

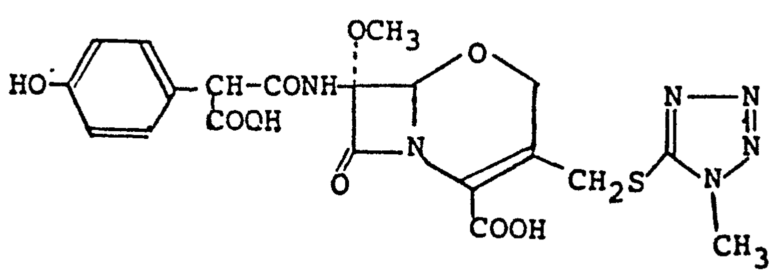

(V) ,

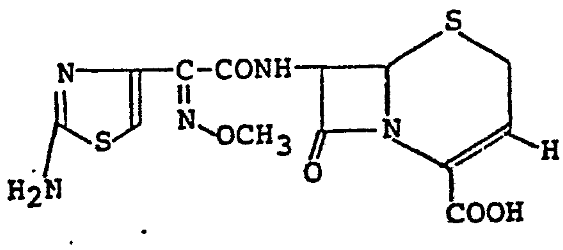

(VI) ,

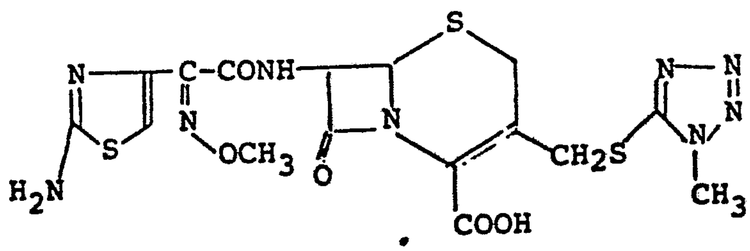

(VII),

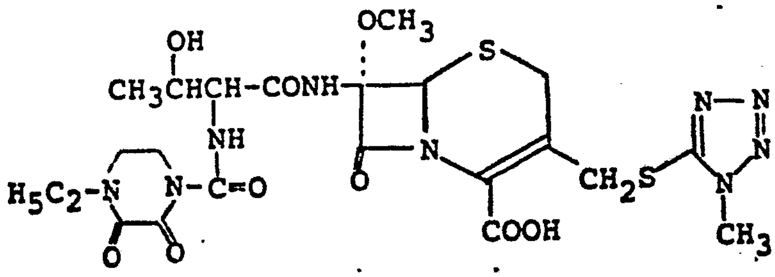

(VIII),

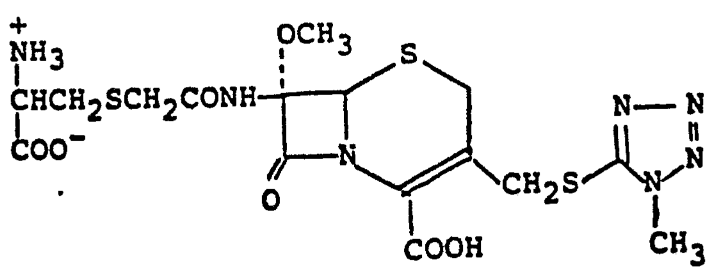

(IX) ,

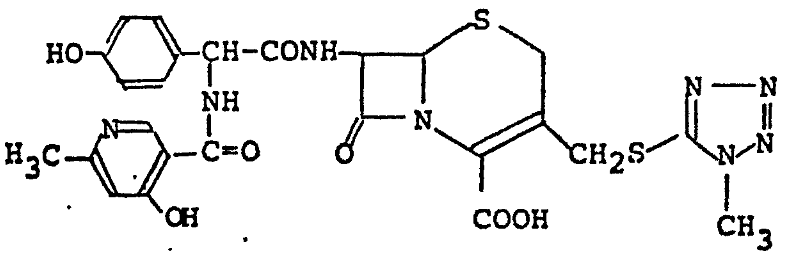

(X),

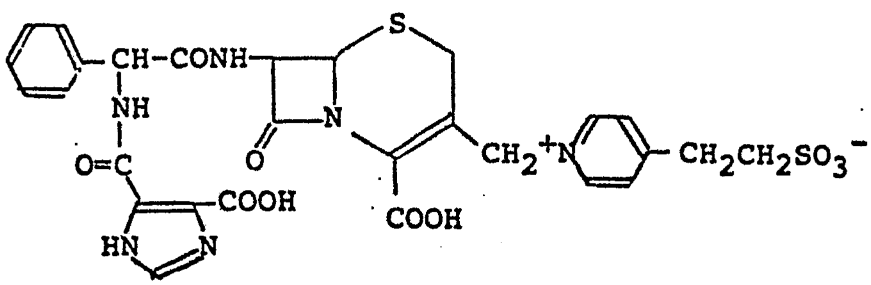

(XI),

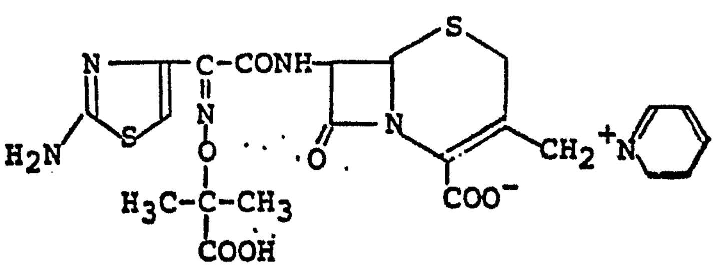

(XII),

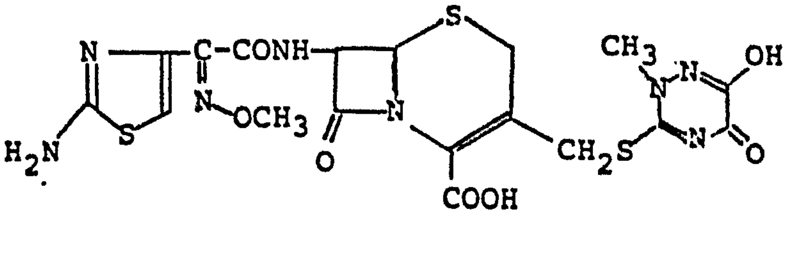

(XIII),

and

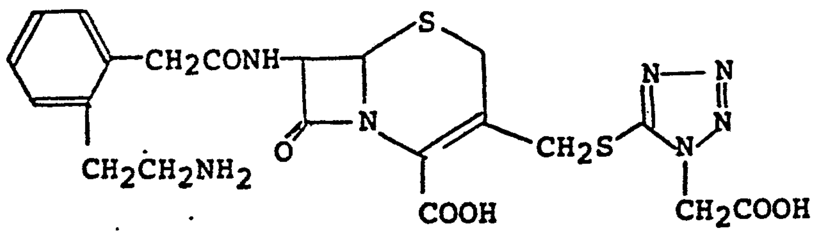

(XIV)

0091502

European Patent Office

# EUROPEAN SEARCH REPORT

Application number

EP 82 10 3114

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | FR-A-2 275 214 (TAKEDA)<br>* pages 17-20; claims * | 1 | A 61 K 31/545<br>A 61 K 31/535<br>A 61 K 9/02 |
| A | GB-A-1 462 399 (TOYAMA)<br>* the whole document * | 1 | |
| A | FR-A-2 035 119 (PLIZER)<br>* the whole document * | 1 | |
| A | CHEMICAL ABSTRACTS, vol.96, no.14, April 5, 1982, page 386, abstract no. 110124h, Columbus, Ohio (US)<br>& JP - A - 81 104 812 (SANKYO CO., LTD.) (August 20, 1981) | 1 | |
| | | | TECHNICAL FIELDS SEARCHED (Int. Cl. 3) |
| A | CHEMICAL ABSTRACTS, vol.96, no.22, May 31, 1982, page 419, abstract no. 187293v, Columbus, Ohio (US)<br>& JP - A - 82 11 917 (DAIICHI SEIYAKU CO., LTD.) (January 21, 1982) | 1 | A 61 K 31/00<br>A 61 K 9/00 |

The present search report has been drawn up for all claims

| Place of search | Date of completion of the search | Examiner |
|---|---|---|
| THE HAGUE | 11-01-1983 | LUYTEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82